(19) **Europäisches Patentamt / European Patent Office / Office européen des brevets**

(11) **EP 3 387 436 B1**

(12) ## FASCICULE DE BREVET EUROPEEN

(45) Date de publication et mention
de la délivrance du brevet:
**06.10.2021 Bulletin 2021/40**

(21) Numéro de dépôt: **16825476.1**

(22) Date de dépôt: **09.12.2016**

(51) Int Cl.:
*G01N 33/50* (2006.01)     *C12Q 1/48* (2006.01)
*G01N 33/573* (2006.01)

(86) Numéro de dépôt international:
**PCT/FR2016/053322**

(87) Numéro de publication internationale:
**WO 2017/098190 (15.06.2017 Gazette 2017/24)**

(54) **MÉTHODE PRÉDICTIVE RAPIDE POUR CARACTÉRISER LA RADIOSENSIBILITÉ D'UN PATIENT ENVERS UNE IRRADIATION AVEC UN RAYONNEMENT IONISANT**

SCHNELLES PRÄDIKTIVES VERFAHREN ZUR CHARAKTERISIERUNG DER STRAHLUNGSEMPFINDLICHKEIT EINES PATIENTEN GEGENÜBER EINER BESTRAHLUNG MIT IONISIERENDER STRAHLUNG

RAPID PREDICTIVE METHOD FOR CHARACTERISING THE RADIOSENSITIVITY OF A PATIENT TO IRRADIATION WITH IONISING RADIATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.12.2015 FR 1502581
28.12.2015 FR 1563362**

(43) Date de publication de la demande:
**17.10.2018 Bulletin 2018/42**

(73) Titulaires:
• **Neolys Diagnostics
67960 Entzheim (FR)**
• **Institut National de la Santé et de la Recherche Médicale
75013 Paris (FR)**
• **Centre Léon Bérard
69008 Lyon (FR)**
• **Centre National de la Recherche Scientifique
75794 Paris Cedex 16 (FR)**
• **Université Claude Bernard Lyon 1
69622 Villeurbanne (FR)**

(72) Inventeurs:
• **FORAY, Nicolas
38300 Meyrie (FR)**
• **GRANZOTTO, Adeline
69008 Lyon (FR)**

• **DEVIC, Clément
69003 Lyon (FR)**
• **FERLAZZO, Mélanie
73400Ugine (FR)**
• **BODGI, Larry
69003 Lyon (FR)**
• **PEREIRA, Sandrine
13650 Meyrargues (FR)**

(74) Mandataire: **IP Trust
2, rue de Clichy
75009 Paris (FR)**

(56) Documents cités:
**WO-A1-2014/184498    WO-A1-2015/121596
WO-A1-2015/121597**

• **GRANZOTTO ADELINE ET AL: "Influence of Nucleoshuttling of the ATM Protein in the Healthy Tissues Response to Radiation Therapy: Toward a Molecular Classification of Human Radiosensitivity", INTERNATIONAL JOURNAL OF RADIATION: ONCOLOGY BIOLOGY PHYSICS, PERGAMON PRESS, USA, vol. 94, no. 3, 14 novembre 2015 (2015-11-14), pages 450-460, XP029398698, ISSN: 0360-3016, DOI: 10.1016/J.IJROBP.2015.11.013**

EP 3 387 436 B1

**Description**

**Domaine technique de l'invention**

L'invention concerne le domaine de la radiobiologie médicale, et plus particulièrement le domaine des méthodes de laboratoire radiobiologiques. L'invention concerne une nouvelle méthode prédictive de la radiosensibilité cellulaire, tissulaire et clinique, qui se base sur la détermination et le recoupement de plusieurs paramètres et critères cellulaires et enzymatiques.

**Etat de la technique**

Environ 1 à 15% des patients traités par radiothérapie pour un cancer montrent une réaction tissulaire (tel qu'une dermite ou une rectite) qui peut mettre en jeu le bon déroulement du traitement dans la mesure où elle peut conduire le médecin à décider l'arrêt du traitement radiothérapeutique avant la fin du protocole prévu. Par ailleurs, cette réaction tissulaire est un indicateur d'une sensibilité particulièrement élevée du patient au rayonnement ionisant. Ainsi, le traitement radiothérapeutique, même interrompu au moment de l'apparition des premiers signes tissulaires visibles, peut augmenter la morbidité voire la mortalité post-traitement des patients, non seulement parce que le cancer qu'il était censé traiter n'a pas pu être éradiqué complètement dû à l'arrêt prématuré du traitement, mais encore à cause de l'endommagement collatéral des tissus sains induit par le rayonnement lui-même.

On sait également que la question de la sensibilité des tissus au rayonnement ionisant est inséparable de celles des mécanismes de réparation des dommages de l'ADN. En effet, au niveau cellulaire, le rayonnement ionisant peut casser certains types de liaisons chimiques en générant des radicaux libres (en particulier par peroxydation) et d'autres espèces réactives à l'origine des dommages de l'ADN. L'endommagement de l'ADN par des agressions endogènes ou exogènes (telles que les radiations ionisantes et les radicaux libres), peut aboutir à différents types de dommages de l'ADN en fonction notamment de l'énergie déposée : les dommages de bases, les cassures simple-brin et les cassures double-brin (CDB). Les CDB non réparées sont associées à la mort cellulaire, la toxicité et plus spécifiquement la radiosensibilité. Les CDB mal réparées sont associées à l'instabilité génomique, aux phénomènes mutagènes et à la prédisposition au cancer. L'organisme dispose de systèmes de réparation spécifiques à chaque type de dommage de l'ADN. En ce qui concerne les CDB, les mammifères possèdent deux principaux modes de réparation : la réparation par suture (ligation des brins) et la réparation par recombinaison (insertion d'un brin homologue ou non-homologue).

On sait que la sensibilité des tissus au rayonnement ionisant est très variable d'un organe à l'autre et d'un individu à l'autre ; l'idée d'une « radiosensibilité intrinsèque » a été conceptualisée par Fertil et Malaise en 1981. Ainsi, les diverses études sur les effets thérapeutiques et les effets secondaires de la radiothérapie ont montré qu'il existe des individus qui jouissent d'une radiorésistance particulièrement élevée, et des individus qui montrent au contraire une radiosensibilité qui peut aller d'un effet secondaire reconnu cliniquement mais sans conséquence jusqu'à un effet létal. Même en dehors de certains rares cas de radiosensibilité extrême, dont l'origine génétique semble avérée, la radiosensibilité semble découler d'une manière générale d'une prédisposition génétique : elle est donc propre à un individu. Il serait donc désirable de disposer d'une méthode d'essai prédictive pour pouvoir déterminer la dose maximale cumulée qu'un patient donné peut recevoir sans risque. Cette question se pose en premier lieu en radiothérapie dans un contexte de fortes doses ionisantes. Toutefois, cette question est également susceptible de se poser pour toute autre exposition à de fortes doses ionisantes, équivalentes à celles utilisées en radiothérapie.

On sait que deux protéines de la famille des kinases, appelées couramment ATM et ATR, sont impliquées dans la détection, la réparation et la signalisation des CDB ; leur action nécessite au moins la présence d'une protéine connue sous la désignation BRCA1 et d'une cascade de phosphorylations ordonnée des différents substrats d'ATM (voir l'article de N. Foray et al., « A subset of A TM- and ATR-dependent phosphorylation events requires the BRCA1 protein », paru dans The EMBO Journal vol. 22(11), p. 2860-2871 (2003)). L'essai a été entrepris d'utiliser l'enzyme ATM dans un modèle explicatif de la radiosensibilité cellulaire (voir Joubert et al., "DNA double-strand break repair defects in syndromes associated with acute radiation response ; At least two different assays to predict intrinsic radiosensitivity ?", paru dans Int. J. Radiat. Biol., vol. 84(2), p. 107-125 (2008)), et cela a permis d'identifier trois types de radiosensibilité : les cellules radiorésistantes (radiosensibilité dite de Groupe I), les cellules modérément radiosensibles (radiosensibilité dite de Groupe II), et les cellules extrèmement radiosensibles (radiosensibilité dite de Groupe III). Cependant, aucun modèle prédictif n'a été proposé sur cette base. De plus Selon notre modèle, le cytoplasme agirait comme un « réservoir » pour des dimères transphosphorylables de la protéine ATM. La protéine se monomérise sous l'action du stress oxydant et c'est sous cette forme monomérique qu'elle serait transportée dans le noyau.

La quasi-totalité des cellules du groupe II ont un nucleo-shuttling d'ATM absent ou retardé après irradiation.

De nombreux documents décrivent les conditions dans lesquelles l'ATM peut contribuer à la détection et la réparation de l'endommagement de l'ADN (Gately et al « Characterization of ATM Expression, Localization, and Associated DNA-dependent Protein Kinase Activity» Molecular biology of the cell 1998 ; Khalil et al « ATM in focus: A damage sensor and cancer target » biodiscovery, 2012 ; Bhatti et al « ATM protein kinase: the linchpin of cellular defenses to stress » Cellular and Molecular Life Science, 2011 ; Khoronenkova et Dianov, « ATM prevents DSB formation by coordinating SSB repair and cell cycle progression », PNAS 2014).

Ces derniers documents décrivent donc des voies de réparation mais n'offrent aucune corrélation pour établir un lien avec la clinique. La demande WO 2015 / 121 596 A1 décrit une méthode prédictive permettant de caractériser la radiosensibilité et la réaction tissulaire d'un patient envers un rayonnement ionisant thérapeutique. Plus particulièrement cette méthode inclut l'irradiation d'un échantillon biologique, la détection et la quantification de foci nucléaires radioinduits sur du tissu conjonctif de type fibroblastique par l'utilisation d'au moins deux marqueurs de détection parmi pH2AX, pATM et MRE11. C'est la seule méthode de l'état de la technique qui permet de quantifier la radiosensibilité individuelle, d'évaluer le risque de réactions tissulaires post-radiothérapie, et qui puisse être employé pour tout patient et tout type de rayonnement ionisant susceptible d'induire des CDB, et qui soit prédictif. Toutefois, l'analyse des focis dans des cellules de type lymphocyte est assez difficile du fait de leur petit noyau et rend cette méthode complexe et chronophage. On a donc souhaité trouver une méthode plus facile à utiliser.

La présente invention vise à proposer une nouvelle méthode prédictive rapide et simple de la radiosensibilité individuelle permettant d'effectuer un criblage des patients et de les orienter si le test est positif vers une méthode plus complète de la radiosensibilité tissulaire et clinique.

**Objets de l'invention**

Les inventeurs ont constaté, et la méthode selon l'invention part de ce constat, que le marqueur pATM renseigne sur l'activation de la voie de suture par phosphorylation de H2AX et l'inhibition de la voie MRE11-dépendante.

Les inventeurs ont par ailleurs observé un transfert des formes cytoplasmiques de la protéine ATM dans le noyau cellulaire à la suite d'un stress de type oxydatif, et notamment à la suite d'un stress lié à un rayonnement ionisant induisant des CDB.

De plus, les inventeurs ont constaté que la quantité de pATM présente dans le cytoplasme et le noyau d'une cellule est fonction du type cellulaire étudié.

Pour constater l'endommagement d'un ADN par une agression exogène, il faut tenir compte, d'une part, de l'état spontané de l'ADN, et, d'autre part, de son état radioinduit. Par ailleurs, après irradiation, il faut tenir compte de la localisation cellulaire de la protéine ATM qui va être directement corrélée à une radiosensibilité. On sait par ailleurs que l'efficacité et la rapidité de la réparation de l'ADN varie d'un individu à l'autre, et qu'il existe en outre des conditions génétiques particulières qui conduisent à une radiosensibilité exceptionnelle. Selon l'invention le problème est résolu par une méthode basée sur : 1) la détection de la quantité de protéine ATM cytoplasmique et nucléaire ; 2) un modèle mécanistique valable pour les cellules humaines notamment des cellules sanguines; 3) des tests fonctionnels quelle que soit la modalité thérapeutique, i.e. le traitement du patient par radiothérapie.

L'objet de l'invention est un procédé pour caractériser la radiosensibilité d'un échantillon cellulaire d'un patient envers un rayonnement ionisant, ledit échantillon cellulaire ayant été obtenu à partir de cellules prélevées sur un patient, dans lequel procédé :

a. on isole des cellules nucléées cibles constituant l'échantillon cellulaire ;
b. on irradie ledit échantillon cellulaire avant ou après l'étape a) avec une dose absorbée D d'un rayonnement ionisant,
c. on récupère les fractions cytoplasmiques et nucléaires de l'échantillon cellulaire ;
d. on détermine sur les fractions cytoplasmiques et/ou nucléaires dudit échantillon cellulaire irradié :

d1. la quantité de substrat phosphorylé par la protéine ATM activée, qui est une protéine cible phosphorylée par la kinase ATM, à au moins un temps d'observation t compris entre t1 fois 1,2 et t3, de préférence à au moins un temps d'observation t2 après irradiation avec une dose absorbée D ; et/ou
d2 . l'activité kinase par la quantification du pATM phosphorylée à au moins un temps d'observation t sélectionné parmi t = t1, t2 ;

e. on détermine au moins un paramètre sélectionné dans le groupe formé par :

- le grade de sévérité de la réaction tissulaire post-radiothérapie du patient selon la classification CTCAE, en utilisant au moins le nombre moyen pATM $_{max\,nuc}$ (t);
- la radiosensibilité de l'échantillon cellulaire en utilisant au moins le nombre moyen pATM $_{max\,nuc}$ (t);

et où

- t1 est une valeur fixe qui représente le temps au bout duquel le nombre de cassures double-brin (CDB) reconnues atteint son maximum dans des cellules témoins issues de patients radiorésistants ;
- t2 est une valeur fixe qui représente le temps au bout duquel environ 50 % des CDB sont réparées dans des fibroblastes témoins issus de patients radiorésistants et le temps au bout duquel 30 % des CDB sont réparées dans des lymphocytes ou lymphoblastes témoins issus de patients radiorésistants;
- t3 est une valeur fixe qui représente z fois t2, où z est compris entre 1,1 et 8, et de préférence compris entre 1,2 et 6.

Les valeurs de z définies ci-dessus sont valables pour tout type de cellules.

Avantageusement, dans ce premier objet de l'invention, on détermine sur les fractions cytoplasmiques et/ou nucléaires dudit échantillon cellulaire irradié soit :

d1. la quantité de protéine cible phosphorylée par la protéine ATM activée à au moins un temps d'observation t compris entre t1 fois 1,2 et t3, de préférence à au moins un temps d'observation t2 après irradiation avec une dose absorbée D, soit

d2. l'activité kinase par la quantification du pATM phosphorylée à au moins un temps d'observation t sélectionné parmi t = t1, t2.

Un objet de la divulgation est un procédé pour caractériser la radiosensibilité d'un échantillon cellulaire d'un patient envers un rayonnement ionisant, ledit échantillon cellulaire ayant été obtenu à partir de cellules prélevées sur un patient, dans lequel procédé:

a. on isole des cellules nucléées cibles constituant l'échantillon cellulaire ;
b. on irradie ledit échantillon cellulaire avant ou après l'étape a) avec une dose absorbée D d'un rayonnement ionisant,
c. on récupère les fractions cytoplasmiques et nucléaires de l'échantillon cellulaire ;
d2. on détermine sur les fractions cytoplasmiques et/ou nucléaires dudit échantillon cellulaire irradié, l'activité kinase par la quantification du pATM phosphorylée à au moins un temps d'observation t,
e. on détermine au moins un paramètre sélectionné dans le groupe formé par :

- le grade de sévérité de la réaction tissulaire post-radiothérapie du patient selon la classification CTCAE, en utilisant au moins le nombre moyen pATM max nuc (t);
- la radiosensibilité de l'échantillon cellulaire en utilisant au moins le nombre moyen pATM max nuc (t);

et où

- t est une valeur fixe qui représente le temps au bout duquel le nombre de CDB reconnues atteint son maximum dans des cellules témoins issues de patients radiorésistants.

Avantageusement, selon cet aspect de la divulgation l'échantillon cellulaire est issu d'une biopsie. Encore plus préférentiellement, selon le second objet de l'invention, les dites-cellules nucléées cibles sont des fibroblastes.

Dans un premier mode de réalisation, à l'étape d1 la quantité du substrat phosphorylé (PP) par la protéine ATM activée est déterminée par spectrométrie de masse. Avantageusement,:

i. on considère que l'échantillon cellulaire est radiorésistant, si à un instant d'observation t compris entre t1 fois 1,2 et t3, et préférentiellement à t2, la relation $qPP_{nuc} \geq qPP_{cyto}$ est valable,

ii. on considère que l'échantillon est radiosensible, si à un instant d'observation t compris entre t1 fois 1,2 et t3, et préférentiellement à t2, la relation $qPP_{cyto} > qPP_{nuc}$ est valable.

où

- $qPP_{cyto}$ correspond à la quantité de peptides phosphorylés dans la fraction cytoplasmique de l'échantillon cellulaire,
- qPPnuc correspond à la quantité de peptides phosphorylés dans la fraction nucléaire de l'échantillon cellulaire,
- PP se réfère au peptide phosphorylé par la protéine ATM ou au substrat phosphorylé par la protéine ATM,
- NPP se réfère au peptide non phosphorylé par la protéine ATM ou au substrat non phosphorylé par la protéine ATM,
- nuc se réfère à la partie nucléaire,
- cyto se réfère à la partie cytoplasmique,
- et q représente un nombre ou une quantité.

Dans un deuxième mode de réalisation avantageux, la quantité de la protéine pATM activée est détectée dans le cytoplasme et le noyau cellulaire par un test ELISA.

Ces deux modes de réalisation sont adaptés à tout type d'échantillon cellulaire, mais de manière avantageuse, l'échantillon cellulaire est choisi parmi : un échantillon sanguin comprenant des lymphocytes, ou des lymphoblastes et un échantillon issu d'une biopsie comprenant des fibroblastes.

Avantageusement, les dites-cellules nucléées cibles sont choisies parmi : des lymphoblastes, des lymphocytes et des fibroblastes.

Dans un autre mode de réalisation avantageux, la radiosensibilité d'un échantillon cellulaire d'un patient envers un rayonnement ionisant est déterminée sur des lymphoblastes et des lymphocytes issus de cellules sanguines de la manière suivante :

- on considère que l'échantillon est radiorésistant si pATM $_{max\,nuc}$ ≥ 0,3 ng par million de cellules,
- on considère que l'échantillon est radiosensible si pATM $_{max\,nuc}$ < 0,3 ng par million de cellules.

où pATM$_{max\,nuc}$ représente la quantité nucléaire maximale en ng de pATM par million de cellules.

Dans un autre mode de réalisation avantageux, la radiosensibilité d'un échantillon cellulaire d'un patient envers un rayonnement ionisant est déterminée sur des fibroblastes issus de biopsies de patient de la manière suivante :

- on considère que l'échantillon est radiorésistant si pATM $_{max\,nuc}$ ≥ A ng par million de cellules,
- on considère que l'échantillon est radiosensible si pATM $_{max\,nuc}$ < A ng par million de cellules.

où pATM$_{max\,nuc}$ représente la quantité nucléaire maximale en ng de pATM par million de cellules et A est une valeur entière ou décimale comprise entre 0,1 et 15, de préférence entre 0,2 et 10 et encore plus préférentiellement entre 0,2 et 5.

Dans un autre mode de réalisation alternatif et avantageux, la radiosensibilité d'un échantillon cellulaire d'un patient envers un rayonnement ionisant est déterminée sur des fibroblastes issus de biopsies de patient de la manière suivante :

- on considère que l'échantillon est radiorésistant si % pATM $_{max\,nuc}$ ≥ B %
- on considère que l'échantillon est radiosensible si % pATM $_{max\,nuc}$ < B %

où % pATM$_{max\,nuc}$ représente la quantité nucléaire maximale en ng de pATM divisée par la quantité totale de protéines cellulaires de l'échantillon X 100 et B est une valeur entière ou décimale comprise entre $0,5 \times 10^{-4}$ et $5 \times 10^{-4}$.

Dans un mode de réalisation avantageux, on détermine le grade de sévérité de la réaction tissulaire post-radiothérapie selon la classification CTCAE selon la formule :

$$grade\ CTCAE = 5 - \frac{C}{10}\left(1 - \exp(-D * pATM_{max\,nuc})\right) \qquad (4)$$

où
pATM$_{max\,nuc}$ représente la quantité nucléaire maximale en ng de pATM par million de cellules. C est une constante entière ou décimale qui représente la quantité maximale de foci pATM$_{max\,nuc}$ atteinte pour un échantillon radiorésistant, notamment observé par immunofluorescence, et qui appartient à l'intervalle [25 ; 60] foci, de préférence qui appartient à l'intervalle [30 ; 50] foci.

D est une constante d'ajustement entière ou décimale qui appartient à l'intervalle [10 ; 30] et qui correspond à l'inverse d'une quantité de pATM en ng par million de cellules.

Dans un mode de réalisation avantageux, on détermine le grade CTCAE du patient à partir de la teneur en pATM$_{max\,nuc}$ (exprimée en ng par million de cellules dans des fibroblastes) selon au moins une des relations suivantes :

| Grades CTCAE | pATM$_{max\,nuc}$ (en ng par million de cellules) |
|---|---|
| 0-1 | ≥0,25 |
| 2-3 | ≥0,07 et <0,25 |
| 4 | ≥0,025 et <0,07 |
| 5 | <0,025 |

Dans un mode de réalisation, on détermine le grade de sévérité de la réaction tissulaire post-radiothérapie selon la classification CTCAE selon la formule :

$$grade\ CTCAE = 5 - 3.561(1 - \exp(-18.6 * pATM_{\max\ nuc})$$

où : $pATM_{max\ nuc}$ représente la quantité nucléaire maximale en ng de pATM par million de cellules issues d'une biopsie, telles que des fibroblastes.

L' objet de l'invention est un procédé pour caractériser la radiosensibilité d'un échantillon cellulaire d'un patient envers un rayonnement ionisant, ledit échantillon cellulaire ayant été obtenu à partir de cellules prélevées sur un patient, dans lequel procédé:

a. on isole des cellules nucléées cibles constituant l'échantillon cellulaire ;
b. on irradie ledit échantillon cellulaire avant ou après l'étape a) avec une dose absorbée D d'un rayonnement ionisant,
c. on récupère les fractions cytoplasmiques et nucléaires de l'échantillon cellulaire ;
d. on détermine sur les fractions cytoplasmiques et/ou nucléaires dudit échantillon cellulaire irradié :

d1. la quantité de protéine cible phosphorylée par la protéine ATM activée à au moins un temps d'observation t compris entre t1 fois 1,2 et t3, de préférence à au moins un temps d'observation t2 après irradiation avec une dose absorbée D, et/ou
d2 . l'activité kinase par la quantification du pATM phosphorylée à au moins un temps d'observation t sélectionné parmi t = t1, t2,

e. on détermine au moins un paramètre sélectionné dans le groupe formé par :

- le grade de sévérité de la réaction tissulaire post-radiothérapie du patient selon la classification CTCAE, en utilisant au moins le nombre moyen pATM $_{max\ nuc}$ (t);
- la radiosensibilité de l'échantillon cellulaire en utilisant au moins le nombre moyen pATM $_{max\ nuc}$ (t);

et où

• t1 est une valeur fixe qui représente le temps au bout duquel le nombre de cassures double-brin (CDB) reconnues atteint son maximum dans des cellules témoins issues de patients radiorésistants ;

• t2 est une valeur fixe qui représente le temps au bout duquel environ 50 % des CDB sont réparées dans des fibroblastes témoins issus de patients radiorésistants et le temps au bout duquel 30 % des CDB sont réparées dans des lymphocytes ou lymphoblastes témoins issus de patients radiorésistants;

• t3 est une valeur fixe qui représente z fois t2, où z est compris entre 1,1 et 8, et de préférence compris entre 1,2 et 6.

Avantageusement, dans cet objet selon l'invention, on détermine sur les fractions cytoplasmiques et/ou nucléaires dudit échantillon cellulaire irradié soit :

d1. la quantité de protéine cible phosphorylée par la protéine ATM activée à au moins un temps d'observation t compris entre t1 fois 1,2 et t3, de préférence à au moins un temps d'observation t2 après irradiation avec une dose absorbée D, soit
d2 . l'activité kinase par la quantification du pATM phosphorylée à au moins un temps d'observation t sélectionné parmi t = t1, t2.

Dans ce mode de réalisation, à l'étape d1 la quantité du protéine cible phosphorylée par la protéine ATM activée est déterminée par spectrométrie de masse.

Dans un autre mode de réalisation, on détermine la radiosensibilité de l'individu selon la méthode du test ELISA sur des lymphoblastes et des lymphocytes issus d'un échantillon sanguin:

(a) Si pour l'échantillon cellulaire $pATM_{max\ nuc}$ < 0,3 ng alors l'échantillon est radiosensible, avec $pATM_{max\ nuc}$ représentant la quantité nucléaire maximale (en ng de pATM par million de cellules),
(b) Si pour l'échantillon cellulaire $pATM_{max\ nuc}$ ≥ 0,3 ng alors l'échantillon est radiorésistant avec $pATM_{max\ nuc}$ représentant la quantité nucléaire maximale (en ng de pATM par million de cellules).

La détermination de la quantité d'ATM activée fait avantageusement intervenir un test protéomique.
Dans un mode de réalisation avantageux, on détermine le grade de sévérité de la réaction tissulaire post-radiothérapie selon la classification CTCAE selon la formule suivante :

$$grade\ CTCAE = 5 - \frac{C}{10}\big(1 - \exp(-D * pATM_{max\ nuc})\big) \qquad (4)$$

où

- pATM$_{max\ nuc}$ représente la quantité nucléaire maximale en ng de pATM par million de cellules issues d'une biopsie,
- C est une constante entière ou décimale qui représente la quantité maximale de foci pATM$_{max\ nuc}$ atteinte pour un échantillon radiorésistant, notamment observé par immunofluorescence, et qui appartient à l'intervalle [25 ;60] foci, de préférence qui appartient à l'intervalle [30 ; 50] foci,
- D est une constante d'ajustement entière ou décimale qui appartient à l'intervalle [10 ; 30] et qui correspond à l'inverse d'une quantité de pATM en ng par million de cellules.

Dans un mode de réalisation avantageux, on détermine les intervalles de pATM$_{max\ nuc}$ (en ng par million de cellules dans des fibroblastes) et les grades CTCAE correspondants selon le tableau suivant :

| Grades CTCAE | pATM$_{max\ nuc}$ (en ng par million de cellules) |
|---|---|
| 0-1 | $\geq$0,25 |
| 2-3 | $\geq$0,07 et <0,25 |
| 4 | $\geq$0,025 et <0,07 |
| 5 | <0,025 |

Dans un autre mode de réalisation lesdites cellules témoins issues de patients radiorésistants ont été sélectionnées en tant que cellules prélevées sur des patients n'ayant pas montré de réactions tissulaires significatives pendant ou à la suite d'un traitement radiothérapeutique.

Le procédé selon l'invention utilise au moins un échantillon de tissu sain, de préférence des fibroblastes ou lymphoblastes/lymphocytes. Les premiers sont de préférence prélevés sur le tissu conjonctif du patient et les deuxièmes dans le sang. Ce prélèvement peut être effectué par biopsie ou prélèvement sanguin.

Ledit rayonnement ionisant est défini par sa dose absorbée (paramètre appelé D et exprimé en Gray). Dans le cadre de la présente divulgation la dose absorbée D est comprise entre 0,5

Gy et 6 Gy, de préférence comprise entre 1 Gy et 6 Gy, de préférence comprise entre 1 Gy et 3 Gy, de préférence entre 1,7 Gy et 2,3 Gy, et est encore plus préférentiellement de 2 Gy. Ces zones correspondent typiquement à une séance individuelle de traitement radiothérapeutique, le nombre de séances dépendant de la localisation, du type et du stade d'avancement de la tumeur.

Il est essentiel pour la méthode selon l'invention que toutes les valeurs de temps t0, t1, t2, soient définies en début d'une série d'essais (i.e. au moins pour un patient donné, et de préférence pour une pluralité de patients afin de calibrer la méthode par rapport à un ensemble d'observations statistiquement significatifs) et soient les mêmes pour toutes les déterminations de tous les paramètres qui se réfèrent à ces intervalles de temps.

Dans une variante du procédé qui est particulièrement intéressante et facile à standardiser, pour les lymphoblastes et lymphocytes t1 est de 5 min et t2 de 10 min, pour les fibroblastes t0 correspond au temps t sans irradiation, t1 est de 10 min post-irradiation, t2 est de 1h post-irradiation et pour les deux types cellulaires D est 2 Gy.

**Description des figures**

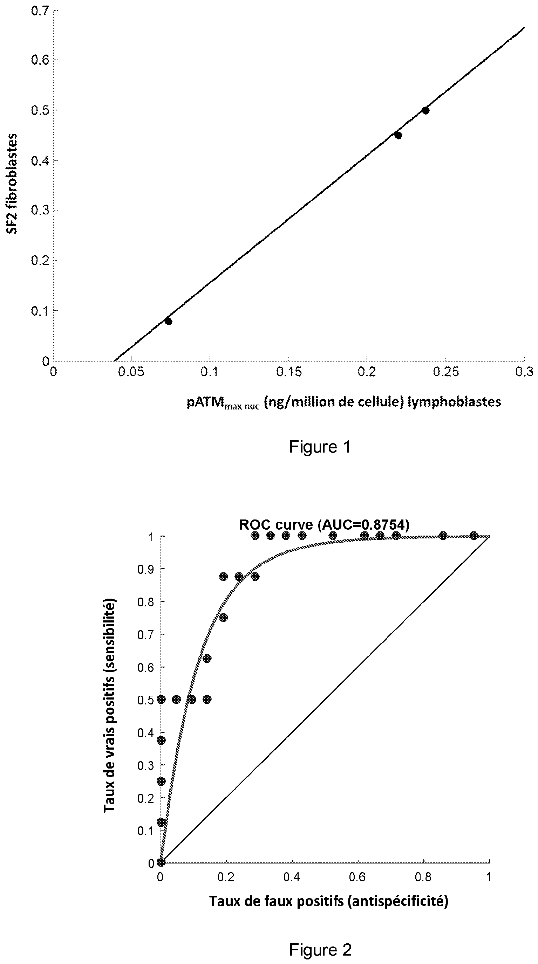

Les figures 1 à 3 illustrent certains aspects de l'invention, mais ne limitent pas sa portée.

La figure 1 montre la corrélation entre la Fraction Survivante à 2 Gy (SF2) observée dans des fibroblastes et la quantité maximale nucléaire de pATM par million de cellules en ng (pATM$_{max\ nuc}$) quantifiée dans les lymphoblastes des mêmes patients.

La figure 2 est un diagramme ROC (de l'anglais Receiver Operating Characteristic) appelé aussi une courbe sensibilité/spécificité. Ce diagramme permet d'évaluer la performance d'un test en représentant le taux de vrais positifs (fraction des patients détectés positifs qui sont détectés correctement par ce test) en fonction du taux de faux positifs (fraction des patients détectés positifs par ce test alors qu'ils sont en réalité négatifs). Ce diagramme montre les performances de la prédiction de la radiosensibilité d'un individu évaluée à partir de fibroblastes selon la méthode du test ELISA explicité ultérieurement où l'échantillon est considéré radiorésistant si pATM $_{max\ nuc} \geq$ A ng par million de cellules ou radiosensible si pATM $_{max\ nuc}$ < A ng par million de cellules.

Cette valeur de A, entière ou décimale, est ainsi comprise entre 0,1 et 15, de préférence entre 0,2 et 10 et encore plus préférentiellement entre 0,2 et 5.

Cette valeur A seuil est fixée et est déterminée par la courbe ROC (tests statistiques en binaire) en fonction du nombre de vrais ou de faux positifs tolérés, i.e. en fonction de la sensibilité et de la spécificité voulues.

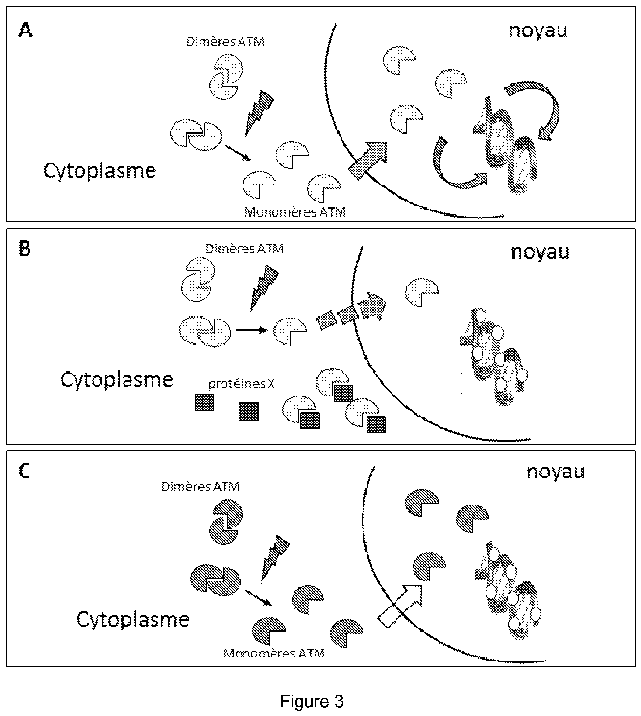

La figure 3 illustre un modèle explicitant le nucleoshuttling de l'ATM radioinduit et les groupes de radiosensibilité. L'ATM est une protéine kinase catalysant les réactions de phosphorylation par l'ajout d'un ion phosphate à une molécule cible appelée substrat, qui est une protéine. Les rayonnements ionisants (IR) induisent l'apparition de cassures double-brin et l'oxydation de l'ATM, provoquant sa monomérisation. La diffusion des monomères ATM permet la reconnaissance de la cassure double-brin et ainsi induit la réparation des CDB. Ainsi, pour des patients radiorésistants, les rayonnements ionisants (IR) vont provoquer la monomérisation de la protéine ATM et sa diffusion dans le noyau cellulaire (cf. figure 3A). Pour des patients de radiosensibilité modérée, un délai est observé dans la diffusion des monomères d'ATM dans le noyau et qui peut être dû à sa réassociation avec d'autres monomères ATM ou des protéines X dans le cytoplasme (cf. figure 3B).

Pour des patients très radiosensibles comme par exemple les patients atteints d'ataxie télangiectasie, la protéine ATM qui est alors inactive ne pourra plus reconnaitre les cassures double-brin (CDB) et ainsi induire la réparation des CDB (cf. figure 3C).

Dans ce contexte, un substrat phosphorylé par la protéine ATM activée à au moins un temps d'observation t après irradiation avec une dose absorbée D correspond à une molécule cible, telle qu'une protéine comprenant un ion phosphate supplémentaire ajouté par un monomère ATM. Un substrat phosphorylé par la protéine ATM activée peut être défini comme étant une protéine cible phosphorylée par la kinase ATM ; c'est dans ce sens qu'est utilisé l'expression protéine cible dans la présente invention.

Les protéines pATM phosphorylées correspondent aux monomères ATM phosphorylés ou activés.

## Description de l'invention

### A. Définitions générales

Les termes « endommagement radioinduit », « radioinduit », « radiosensibilité », « radiorésistance », « radiotoxicité », « radiothérapie » se réfèrent tous à un rayonnement ionisant, notamment à un rayonnement de type particules, tel que constitué par des particules de type alpha ($\alpha$) ou béta ($\beta$), ou encore à un rayonnement électromagnétique à haute énergie, notamment de type gamma ($\gamma$) ou X.

### B. Description détaillée

Nous décrivons ici un mode de réalisation avec plusieurs variantes qui convient pour un patient humain.

#### 1. Préparation de l'essai

Avant tout prélèvement de cellules et avant toute manipulation des cellules prélevées, les opérateurs respectifs (appartenant par exemple à un laboratoire d'analyses cytologiques) sont informés (typiquement par le médecin) du statut d'infection éventuelle du patient par HIV ou hépatite C pour que les opérateurs puissent prendre les mesures appropriées de sécurité biologique accrue lors du prélèvement, de la manipulation et de la gestion de la culture cellulaire.

Puis, l'opérateur prélève au patient un échantillon cellulaire.

Le matériel biologique qui sera irradié peut être notamment des lymphoblastes, des lymphocytes isolés ou des cellules isolées à partir de tissu.

En ce qui concerne les lymphocytes isolés, de manière très préférée ils sont isolés à partir de sang total frais via la technique du ficoll (Lifescience). Cette technique connue de l'homme du métier est utilisée depuis plus de 30 ans et décrite notamment dans l'article de Bøyum, A. 1968 « Isolation of mononuclear cells and granulocytes from human blood. » (Paper IV), Scand. J. Clin. Lab. Invest. 21 Suppl. 97, 77-89 (1968).

Ensuite on expose l'échantillon cellulaire à une irradiation avec une dose de 2 Gy. Les effets sont observés à deux temps précoces post-irradiation, t1 (5 min) et t2 (10 min) pour les lymphocytes (petites cellules) et à des temps différents comprenant un temps t0 (état non irradié) et des temps plus tardifs t1 (10 min) et t2 (1h) pour les fibroblastes.

Pour chaque temps les cellules sont récupérées via une trypsination pour les fibroblastes et une centrifugation pour les lymphocytes.

Ensuite le matériel biologique est soumis à un fractionnement subcellulaire afin de séparer les protéines cytoplasmiques des protéines nucléaires aux différents temps t0, t1 et t2. Dans un mode de réalisation, ce fractionnement est réalisé par le biais d'un kit (PIERCE) selon la méthodologie décrite par exemple dans l'article de Trotter, K. W. and Archer, T. K., Molecular and Cellular Biology (2004) "Reconstitution of glucocorticoid receptor-dependent transcription in vivo ».

Les protéines ATM sont présentes dans chacune des fractions cytoplasmiques et nucléaires. Ce point est d'ailleurs vérifié par un essai de type « Western blot » avec différents anticorps spécifiques de chaque compartiment cytoplasmique (anti-tubuline ou anti-actine) et nucléaire (anti-topoisomérase ).

L'isolation et la capture des protéines ATM dans chacune des fractions cytoplasmiques et nucléaires peuvent être réalisées par immunoprécipitation, et la quantification peut être réalisée par spectrométrie de masse ou par un test ELISA. Nous décrivons ici un mode de réalisation typique pour chacune de ces voies.

a) Méthode de la spectrométrie de masse :

L'isolation et la capture des protéines ATM sous forme de dimères, de monomères phosphorylés ou de monomères non phosphorylés dans chacune des fractions cytoplasmiques et nucléaires peut être réalisée par immunoprécipitation grâce à un anticorps spécifique anti-ATM (AbCam).

L'activité enzymatique de la protéine ATM est ensuite déterminée à l'aide d'un test enzymatique de phosphorylation par la protéine kinase ATM active, d'une protéine cible ou substrat spécifique la protéine DNApK. La protéine ATM sous forme de monomères phosphorylés, i.e. forme active de la protéine ATM réagit avec le substrat du test enzymatique permettant de quantifier la quantité de substrat phosphorylé par la protéine ATM activée et indirectement de déterminer la quantité de protéine ATM activée. Les autres formes non actives de la protéine ATM ne réagissent pas avec le substrat. L'ensemble de la réaction (protéine kinase ATM, substrat DNApK, ATP) est ensuite digéré par une trypsine, séparé par chromatographie et analysé par spectrométrie de masse. Une analyse quantitative des différents éléments est possible et ce grâce à l'emploi de peptides marqués tels que des peptides marqués spécifiques des substrats DNApK phosphorylés ou non phosphorylés, des peptides marqués spécifiques de la protéine kinase ATM, permettant de détecter le nombre de substrats phosphorylés et non phosphorylés mais aussi la quantité de protéine ATM (normalisation des résultats). Ledit marquage spécifique des peptides peut être réalisé par substitution isotopique ou par tout autre moyen qui ne modifie pas la fonction biologique du peptide.

b) Méthode du Test ELISA :

L'isolation et la capture des protéines ATM phosphorylées (donc actives) dans chacune des fractions cytoplasmiques et nucléaires est réalisée par TEST ELISA (Kit R&D systems et Novateinbio) grâce à un anticorps spécifique anti-pATM revêtu dans des plaques (R&D systems et Novateinbio). La quantité de pATM est ensuite analysé par luminescence à 450 nm grâce à un lecteur de plaques.

Cette méthode a été mise en œuvre et validée sur trois lignées de lymphoblastes et 30 lignées de fibroblastes humains. Ces lignées ont été caractérisées par la fraction survivante à 2 Gy (SF2) et aussi en immunofluorescence pour le marquage H2AX et pATM (lignées issues de patients).

2. Détermination des paramètres biologiques et cliniques

2.1 Généralités et marqueurs utilisés

L'invention est basée entre autres sur l'utilisation de données acquises pour le marqueur pATM sur des cellules non-exposées (état spontané) et exposées. La méthode est basée sur l'étude de la quantification de ce marqueur pour une durée post-irradiation donnée : on analyse l'activité kinase de cette protéine ATM dans les fractions cytoplasmiques et nucléaires des échantillons après un laps de temps déterminé à compter de l'arrêt de l'irradiation, et on étudie soit:

- la phosphorylation d'un substrat d'ATM,
- la quantité de la protéine ATM active c'est-à-dire la quantité de pATM.

Les moyennes obtenues pour chaque point et chaque dose avec chaque méthode sont calculées avec les erreurs écart-types de la moyenne (appelé « SEM ») sur un nombre d'échantillons supérieur ou égal à 2. Lorsque le nombre d'échantillons est inférieur à 30, l'écart-type gaussien de type « standard error SE » ne peut être appliqué (règle statistique).

2.2 Paramètres biologiques

On définit et détermine comme indiqué :

pATM$_{max\,nuc}$ est la quantité nucléaire maximale (en ng) de pATM par million de cellules détectée par ELISA.

% pATM$_{max\,nuc}$ représente la quantité nucléaire maximale en ng de pATM divisée par la quantité totale de protéines cellulaires de l'échantillon X 100.

N$_{max}$ pATM est le nombre maximal de foci de pATM.

qPP et qNPP correspondent respectivement aux quantités de peptides phosphorylés par ATM et de peptides non phosphorylés détectés par spectrométrie de masse.

2.3 <u>Evaluation prédictive</u>

Elle vise la prédiction de paramètres cliniques à partir des données biologiques mesurées.

La méthode selon l'invention permet un diagnostic quantitatif, qui est directement issu de la valeur mathématique des scores ou de formules mathématiques reliant les scores.

On décrit ici d'abord la détermination du statut de radiosensibilité (pouvant être réalisée sur lymphocytes, lymphoblastes ou fibroblastes, ici l'exemple des fibroblastes).

Après analyse de la quantité des formes actives de la protéine ATM dans les compartiments cytoplasmiques et nucléaires, on peut déterminer une première approximation des groupes de radiosensiblité par cette méthode pour les lymphoblastes. Ceci implique qu'à partir de d'échantillons de sang, seul le statut de radiosensibilité peut être déterminé.

Selon l'invention, on considère que :

- si pour l'échantillon cellulaire pATM$_{maxnuc}$ < 0,3 ng alors l'échantillon est radiosensible,
- si pour l'échantillon cellulaire pATM$_{maxnuc}$ ≥ 0,3 ng alors l'échantillon est radiorésistant.

Dans ces formules, pATM$_{max\,nuc}$ représente la quantité nucléaire maximale exprimée en ng de pATM par million de cellules.

Ces résultats sont basés sur la mise en évidence d'une première corrélation linéaire entre les résultats de la SF2, de l'immunofluorescence et les quantités maximales de pATM mesurées dans le noyau jusqu'à 10 min post-irradiation dans les lymphoblastes (voir le tableau 1 et la figure 1).

Les patients 40579 et 37572 présentent des mutations sur le gène BRCA1 induisant une tendance à développer des cancers du sein ou de l'ovaire.

Les lignées de lymphoblastes GM07078, 40579 et 37572 correspondent à des patients pour lesquels des expérimentations ont été réalisées à la fois sur lymphoblastes et sur fibroblastes. Ainsi ont été déterminés pour ces patients sur ces deux types cellulaires :

- la Fraction survivante à 2 Gy (SF2),

- le nombre de foci de H2AX.

Ces données entre fibroblastes et lymphoblastes ont été comparées entre elles et une corrélation linéaire a été mise en évidence en ajustant les résultats entre la SF2 des fibroblastes et la quantité maximale de pATM des lymphoblastes.

Dans le tableau 1 sont présentés les résultats de ces 3 patients en termes de survie et de foci de H2AX sur lymphoblastes et fibroblastes avec les données quantitatives de pATM sur lymphoblastes

Tableau 1 :

| Valeurs maximales en ng de la protéine pATM par million de cellules à (pATM$_{max\,nuc}$) dans les noyaux des lymphoblastes et corrélation avec la survie des fibroblastes à une dose de 2 Gy | | | | | |
|---|---|---|---|---|---|
| patient | phénotype | SF2 Lymphoblastes | SF2 Fibroblastes | Nombre de foci H2AX à 24h lymphoblastes | max pATM par million de cellules |
| GM07078 | NBS homoz | 0,04 | 0,08 | 13,7 | 0,07 |
| 40579 | BRAC1 +/- | 0,1 | 0,45 | 5,2 | 0,22 |
| 37572 | BRAC1 +/- | 0,2 | 0,45 | 2,75 | 0,24 |

La figure 1 illustre la corrélation entre la SF2 observée dans des fibroblastes et la quantité maximale nucléaire de pATM par million de cellules en ng (pATM$_{max\ nuc}$) quantifiée dans les lymphoblastes des mêmes patients.

Ces résultats illustrent la validité empirique de la formule suivante :

$$SF2 = K\ pATM_{max\ nuc} - L$$

avec

- K une constante entière ou décimale qui décrit l'évolution de la SF2 en fonction de pATM$_{max\ nuc}$, et qui appartient à l'intervalle [2,3] en millions de cellules par ng,
- et L une constante entière ou décimale qui appartient à l'intervalle [0, 1].

Plus particulièrement, la fraction survivante à 2 Gray sur des fibroblastes peut être déterminée selon la formule suivante :

$$SF2 = 2,55\ pATM_{max\ nuc} - 0,1$$

où pATM$_{max\ nuc}$ désigne la quantité nucléaire maximale en ng de pATM par million de cellules dans des lymphoblastes.

On décrit maintenant la détermination exacte du statut de radiosensibilité d'un patient à partir de fibroblastes (tableaux 2 et 3).

Selon l'invention on considère que :

- on considère que l'échantillon est radiorésistant si pATM$_{max\ nuc}$ ≥ A ng par million de cellules,
- on considère que l'échantillon est radiosensible si pATM$_{max\ nuc}$ < A ng par million de cellules.

où pATM$_{max\ nuc}$ représente la quantité nucléaire maximale en ng de pATM par million de cellules et A est une valeur seuil entière ou décimale comprise entre 0,1 et 15, de préférence entre 0,2 et 10 et encore plus préférentiellement entre 0,2 et 5.

La valeur A est déterminée en fonction de la performance requise pour le test prédictif, notamment par la courbe ROC (tests statistiques en binaire) en fonction du nombre de vrais ou de faux positifs tolérés, i.e. en fonction de la sensibilité et de la spécificité voulues (cf. figure 2). A titre d'exemple, A est environ de 0,46 ng par million de cellules pour une sensibilité de 0,87 et une spécificité de 0,80.

Une méthode alternative consiste à déterminer la radiosensibilité de l'échantillon cellulaire de la manière suivante :

- on considère que l'échantillon est radiorésistant si % pATM$_{max\ nuc}$ ≥ B %
- on considère que l'échantillon est radiosensible si % pATM$_{max\ nuc}$ < B %

où % pATM$_{max\ nuc}$ représente la quantité nucléaire maximale en ng de pATM divisée par la quantité totale de protéines cellulaires de l'échantillon X 100 et B est une valeur entière ou décimale comprise entre $0,5 \times 10^{-4}$ et $5 \times 10^{-4}$.

Cette valeur B seuil est fixée et est déterminée par la courbe ROC qui permet d'évaluer la performance de ce test en représentant le taux de vrais positifs (fraction des patients détectés positifs qui sont détectés correctement par ce test) en fonction du taux de faux positifs (fraction des patients détectés positifs par ce test alors qu'ils sont en réalité négatifs). Cette valeur B seuil est fixée et est déterminée en fonction de la performance requise pour le test prédictif, notamment par la courbe ROC (tests statistiques en binaire) en fonction du nombre de vrais ou de faux positifs tolérés, i.e. en fonction de la sensibilité et de la spécificité voulues.

pATM$_{max\ nuc}$ est la quantité nucléaire maximale (en ng) de pATM par million de cellules détectée par ELISA.

% pATM$_{max\ nuc}$ représente la quantité nucléaire maximale en ng de pATM divisée par la quantité totale de protéines cellulaires de l'échantillon X 100.

La détermination exacte du statut de radiosensibilité d'un patient à partir de fibroblastes à partir d'une valeur seuil exprimée en ng par million de cellules est plus précise que celle effectuée à partir du % pATM$_{max\ nuc}$.

Le grade CTCAE a été déterminé cliniquement (cf. tableaux 2 et 3) en fonction des symptômes des patients. Le statut de radiosensibilité des patients présenté dans les tableaux 2 et 3 suivants, a été déterminé à partir du grade CTCAE déterminé cliniquement, i.e. le patient observé est radiorésistant pour un grade CTCAE clinique inférieur ou égal à 1 et radiosensible pour un grade CTCAE observé cliniquement supérieur ou égal à 2.

A partir de ces données cliniques, pour les fibroblastes, les inventeurs ont trouvé des corrélations entre le nombre maximal de foci de pATM et la quantité maximale de pATM nucléaires dans différentes lignées (syndromes génétiques et lignées issues de biopsies de patients) ; des résultats sont documentés sur les tableaux 2 et 3. Le tableau 2 ci-dessous présente, pour différentes lignées, la quantité nucléaire maximale de pATM en ng par million de cellules permettant de déterminer ultérieurement la valeur seuil entière ou décimale A, puis de déterminer grâce à cette valeur A, le statut de radiosensibilité selon l'invention.

Tableau 2

| Valeurs maximales (en ng) de la protéine pATM par million de cellules (pATM$_{max\,nuc}$) dans les fibroblastes | | | | |
|---|---|---|---|---|
| Lignée | pATM $_{max\,nuc}$ (quantité nucléaire maximale en ng de pATM par million de cellules) | max foci pATM | Grade CTCAE observé cliniquement (nombre entier) | statut de radiosensibilité déterminé cliniquement |
| 02CUR | 0,57 | 35,00 | 1 | radiorésistant |
| 03OLL | 0,38 | 35,00 | 1 | radiorésistant |
| HDF | 2,13 | 40,00 | 0 | radiorésistant |
| HFF2 | 0,52 | -40,00 | 0 | radiorésistant |
| IMR90 | 2,94 | 41,00 | 0 | radiorésistant |
| MRC7 | 1,28 | 40,00 | 0 | radiorésistant |
| MRC9 | 0,89 | 40,00 | 0 | radiorésistant |
| THORS 2 TS1 | 0,6 | -22,00 | 1 | radiorésistant |
| 01 CJP | 0,24 | 16,66 | 3 | radiosensible |
| 01DAX | 0,46 | 23,33 | 3 | radiosensible |
| 01JEM | 0,11 | 28,30 | 2 | radiosensible |
| 02CAV | 0,07 | 20,00 | 3 | radiosensible |
| 02CLB | 0,05 | 15,00 | 4 | radiosensible |
| 02MTZ | 0,06 | 30,00 | 3 | radiosensible |
| 03HLS | 0,05 | 23,30 | 3 | radiosensible |
| 03HM | 0,11 | 31,00 | 2 | radiosensible |
| 04CAV | 0,03 | 18,00 | 4 | radiosensible |
| 05HM | 0,04 | 15,00 | 3 | radiosensible |
| 07HM | 0,57 | 25,00 | 3 | radiosensible |
| 07HNG | 0,16 | 24,00 | 3 | radiosensible |
| 08HNG | 0,04 | 28,00 | 3 | radiosensible |
| 09HM | 0,31 | 33,00 | 2 | radiosensible |
| 10HM | 0,05 | 32,00 | 2 | radiosensible |
| 11HM | 0,67 | 34,00 | 2 | radiosensible |
| 14CLB | 0,1 | 20,00 | 3 | radiosensible |
| 17CLB | 0,1 | 25,00 | 3 | radiosensible |
| 21CLB | 0,39 | 29,50 | 2 | radiosensible |
| 28CLB | 0,87 | 21,00 | 2 | radiosensible |
| 34CLB | 0,72 | 16,67 | 3 | radiosensible |

Tableau 3

| Valeurs en % de $pATM_{max\ nuc}$ pour différentes lignées (% de $pATM_{max\ nuc}$ représente la quantité nucléaire maximale en ng de pATM divisée par la quantité totale de protéines cellulaires de l'échantillon X 100 dans les fibroblastes) | | | | | | |
|---|---|---|---|---|---|---|
| Lignée | %pATM max nuc | SEM | %SEM | max foci pATM | Grade CTCAE observé cliniquement (nombre entier) | statut de radiosensibilité déterminé cliniquement |
| 02 CUR | 2,43E-04 | 1,07E-04 | 44% | 35,00 | 1 | radiorésistant |
| 03 OLL | 9,08E-05 | 2,27E-05 | 25% | 35,00 | 1 | radiorésistant |
| 03 OLL | 1,51E-04 | 3,97E-06 | 3% | 35,00 | 1 | radiorésistant |
| 1 Br3 | 1,57E-04 | 2,05E-05 | 13% | 40,00 | 1 | radiorésistant |
| HDF | 3,21E-04 | 8,23E-05 | 26% | 40,00 | 0 | radiorésistant |
| IMR-90 | 3,19E-04 | 8,17E-05 | 26% | 40,00 | 0 | radiorésistant |
| MRC7 | 2,52E-04 | 1,43E-04 | 57% | 40,00 | 0 | radiorésistant |
| MRC9 | 1,01E-04 | 2,60E-05 | 26% | 40,00 | 0 | radiorésistant |
| 01 CJP | 3,08E-05 | 4,35E-07 | 1% | 16,66 | 3 | radiosensible |
| 01 OLL | 1,15E-04 | 6,30E-05 | 55% | 17,00 | 3 | radiosensible |
| 01DAX | 7,43E-05 | 5,29E-06 | 7% | 23,00 | 3 | radiosensible |
| 02 CLB | 1,29E-04 | 2,17E-05 | 17% | 15,00 | 4 | radiosensible |
| 03 HM | 1,49E-04 | 1,91E-05 | 13% | 31,00 | 2 | radiosensible |
| 05 HM | 1,39E-04 | 5,94E-06 | 4% | 15,00 | 3 | radiosensible |
| 07 HM | 1,92E-04 | 8,10E-06 | 4% | 25,00 | 3 | radiosensible |
| 09 HM | 1,54E-04 | 2,21E-06 | 1% | 33,00 | 2 | radiosensible |
| 10 HM | 9,67E-05 | 7,35E-06 | 8% | 32,00 | 2 | radiosensible |
| 11 HM | 1,02E-04 | 2,84E-05 | 28% | 34,00 | 2 | radiosensible |
| 21CLB | 5,77E-05 | 9,57E-06 | 17% | 29,50 | 2 | radiosensible |
| 03 HLS | 1,92E-04 | 1,28E-04 | 67% | 23,33 | 3 | radiosensible |
| 04 CAV | 1,12E-04 | 3,52E-06 | 3% | 18,00 | 4 | radiosensible |
| 02-MTZ | 2,44E-04 | 1,22E-05 | 5% | 30,00 | 3 | radiosensible |
| 02-CAV | 1,56E-04 | 2,46E-06 | 2% | 20,00 | 3 | radiosensible |
| 34 CLB | 1,54E-04 | 2,21E-06 | 1% | 16,67 | 3 | radiosensible |

Le tableau 3 présente le % de $pATM_{max\ nuc}$, pour différentes lignées, permettant de déterminer le % seuil (B) qui permet ultérieurement de déterminer le statut de radiosensibilité selon l'invention.

On décrit maintenant la détermination des grades de sévérité CTCAE (exemple des fibroblastes).

La classification dite CTCAE (Common Terminology Criteria for Adverse Events, appelée en français « Critères d'évaluation de la morbidité selon la classification du National Cancer Institute»), éditée en 2006 par le National Cancer Institute des Etats Unis d'Amérique, est une terminologie descriptive des événements indésirables (en particulier des effets secondaires) en thérapie du cancer.

Un événement indésirable correspond à tout signe défavorable et involontaire, symptôme ou maladie temporellement associé à l'utilisation d'un traitement médical ou d'une procédure qui peut ou non être considéré comme lié au traitement ou à la procédure médicale. Un événement indésirable est une représentation unique d'un événement spécifique utilisé pour la documentation médicale et lors d'analyses scientifiques.

La CTCAE fournie une brève définition de chaque événement indésirable pour clarifier le sens de l'événement indésirable. Cette échelle, valable pour d'autres stress génotoxique (ex : blessures causées par le feu) est particulièrement utilisée en radiothérapie.

Le grade se réfère à la sévérité de l'événement indésirable. La CTCAE affiche 5 grades de sévérité (de 1 à 5) présentant des descriptions cliniques uniques de sévérité pour chaque événement indésirable et décrits dans le tableau 4 ci-après. Chaque grade de sévérité est défini par des réactions tissulaires précises.

Tableau 4 : dernière version de l'échelle CTCAE éditée par le National Cancer Institute des Etats Unis d'Amérique le 14 juin 2010

| grade 1 | Sévérité légère; symptômes asymptomatiques ou légers; observations cliniques ou diagnostiques seules; intervention non indiquée |
| --- | --- |
| grade 2 | Sévérité modérée; intervention minimale, intervention locale ou non invasive indiquée; événement limitant les activités de la vie quotidienne instrumentées et adaptées à l'âge (préparation du repas, faire les courses, utilisation du téléphone...) |
| grade 3 | Sévérité grave ou médicalement significative, mais ne mettant pas immédiatement la vie en danger; hospitalisation ou prolongation de l'hospitalisation indiquée; événement invalidant; événement limitant les activités de soins de la vie quotidienne (bains, s'habiller, se nourrir, utilisation des toilettes, prise de médicaments, et ne pas être alité) |
| grade 4 | Conséquences potentiellement mortelles; intervention urgente indiquée |
| grade 5 | Décès lié à l'événement indésirable |

A ces 5 grades, est ajouté un grade 0 correspondant à une absence d'effet tissulaire.

Pour les fibroblastes, les inventeurs ont trouvé des corrélations entre le nombre maximal de foci de pATM et la quantité maximale de pATM nucléaires dans différentes lignées (syndromes génétiques et lignées issues de biopsies de patients) ; des résultats sont documentés sur les tableaux 5 et 6.

Tableau 5

| Valeurs maximales (en ng) de la protéine pATM par million de cellules ($pATM_{max\ nuc}$) dans les fibroblastes | | | |
| --- | --- | --- | --- |
| Identification | phénotype | max pATM nucléaire par million de cellules | Nombre max foci pATM |
| Témoin | radiorésistant | 0,27 | 40 |
| A1 | Cancer poumon | 0,105 | 23 |
| GM02718 | Rétinoblastome | 0,124 | 26 |
| AG11498 | progéria | 0,19 | 25 |
| A2 | cancer ORL | 0,069 | 20 |

Les lignées GM02718, AG11498 et les cellules témoins sont les lignées cellulaires issues du Coriell Institute, alors que les lignées A1 et A2 ont été établies à partir de biopsies de patients.

Tableau 6

| Intervalles de $pATM_{max\ nuc}$ en ng par million de cellules et les grades CTCAE correspondants | |
| --- | --- |
| Grades CTCAE | $pATM_{max\ nuc}$ |
| 0-1 | ≥0,25 |
| 2-3 | ≥0,07 et <0,25 |
| 4 | ≥0,025 et <0,07 |
| 5 | <0,025 |

On trouve que le nombre maximal de foci pATM observé par immunofluorescence en fonction de la quantité maximale de pATM nucléaire en ng par millions de cellules obéit à la loi :

$$N_{max}^{pATM} = C(1 - \exp(-D * pATM_{\max nuc})) \quad (1)$$

où $pATM_{\max nuc}$ représente la quantité nucléaire maximale en ng de pATM par million de cellules.

C est une constante entière ou décimale qui représente la quantité maximale de foci $pATM_{\max nuc}$ atteinte pour un échantillon radiorésistant, observé par immunofluorescence, et qui appartient à l'intervalle [25 ; 60] foci, de préférence qui appartient à l'intervalle [30 ; 50] foci.

D est une constante d'ajustement décimale qui appartient à l'intervalle [10 , 30] et qui correspond à l'inverse d'une quantité de pATM en ng par million de cellules.

Selon la demande WO 2015 / 121 596 A1, le grade CTCAE a été défini ici comme suit, en fonction du nombre maximal de foci de pATM obtenu après irradiation avec une dose absorbée D :

$$grade\ CTCAE = 5 - \frac{N_{max}^{pATM}}{10} \quad (2)$$

Sur la base de l'équation (1), le grade de sévérité tissulaire selon la classification CTCAE peut être prédit par la méthode du test ELISA sur fibroblastes en remplaçant $N_{max}^{pATM}$ par sa valeur dans l'équation (2). Le grade de sévérité tissulaire selon la classification CTCAE peut être déterminé selon l'équation suivante :

$$grade\ CTCAE = 5 - \frac{C}{10}(1 - \exp(-D * pATM_{\max nuc})) \quad (3)$$

où

- $pATM_{\max nuc}$ représente la quantité nucléaire maximale en ng de pATM par million de cellules,
- C est une constante entière ou décimale qui représente la quantité maximale de foci $pATM_{\max nuc}$ atteinte pour un échantillon radiorésistant, observé par immunofluorescence, et qui appartient à l'intervalle [25 ; 60] foci, de préférence qui appartient à l'intervalle [30 ; 50] foci.
- D est une constante d'ajustement décimale qui appartient à l'intervalle [10 , 30] et qui correspond à l'inverse d'une quantité de pATM en ng par million de cellules.

Les intervalles de $pATM_{\max nuc}$ en ng par million de cellules et les grades CTCAE correspondants sont indiqués dans le tableau 6.

L'utilisation de l'équation (3) selon l'invention permettant la détermination du grade de sévérité selon la classification CTCAE conduit à l'obtention d'un nombre décimal.

Selon l'invention, la valeur du grade de sévérité tissulaire selon la classification CTCAE est un nombre entier correspondant à l'arrondi arithmétique de la valeur obtenue par calcul. La CTCAE déterminée selon l'invention et indiquée dans la demande correspond à ce nombre entier.

La méthode selon l'invention permet aussi un diagnostic semi-quantitatif directement issu de la valeur mathématique des scores ou de formules mathématiques reliant les scores celui-ci concerne le critère suivant avec la méthode semi-quantitative de la spectrométrie de masse, telle que décrite ci-dessus. Selon l'invention, on considère que pour l'échantillon cellulaire :

- si à un instant d'observation t compris entre t1 fois 1,2 et t3, préférentiellement à t2, $qPP_{nuc} \geq qPP_{cyto}$, on considère que l'échantillon cellulaire est radiorésistant,
- si à un instant d'observation t compris entre t1 fois 1,2 et t3, préférentiellement à t2, $qPP_{cyto} > qPP_{nuc}$, on considère que l'échantillon est radiosensible,

où

- $qPP_{cyto}$ correspond à la quantité de peptides phosphorylés dans la fraction cytoplasmique de l'échantillon cellulaire,
- $qPP_{nuc}$ correspond à la quantité de peptides phosphorylés dans la fraction nucléaire de l'échantillon cellulaire,
- PP se réfère au peptide phosphorylé par la protéine ATM,
- nuc à la partie nucléaire,
- cyto à la partie cytoplasmique,
- et q représente un nombre ou une quantité.

La validation analytique d'une dizaine de lignées a été réalisée à chaque fois avec une corrélation établie avec la détermination du statut de radiosensibilité réalisé par immunofluorescence avec les marqueurs H2AX et pATM selon la méthode décrite dans WO 2015 / 121 596 A1. Ces résultats sont rassemblés dans les tableaux 7 et 8.

Tableau 7 : Résultats qualitatifs obtenus sur des lymphoblastes par spectrométrie de masse

| Lignée testée | Activité ATM quantitatif mesurée par spectrométrie de masse | | Radiosensibilité du patient |
| --- | --- | --- | --- |
| | à t1 = 5 3in | à t2 = 10 min | |
| Témoin GM03798 | $qPP_{nuc} < qPP_{cyto}$ | $qPP_{nuc} > qPP_{cyto}$ où $qPP_{cyto} = 0$ | radiorésistant |
| GM13515 HD (patient atteint de la maladie de Huntington) | $qPP_{nuc} < qPP_{cyto}$ | $qPP_{nuc} < qPP_{cyto}$ | radiosensible |

Dans tous les exemples du tableau 7, ces résultats obtenus ont été confirmés par immunofluorescence.

Tableau 8 : Résultats quantitatifs obtenus sur des lymphoblastes par spectrométrie de masse

| Lignée testée | Activité ATM en % phosphorylation (SM) | | Activité ATM en % phosphorylation (SM) | | Radiosensibilité selon l'invention |
| --- | --- | --- | --- | --- | --- |
| | à t1 =5 min cyto | à t1 =5 min nuc | à t2 =10 min cyto | à t2 =10 min nuc | |
| Témoin lympho GM03798 | 0,3229 | 0,1859 | 0 | 4,7920 | radiorésistant |
| Lympho GM13515 HD (maladie de Huntington) | 0,3586 | 0 | | | radiosensible |
| Témoin fibro 149 BR | | | 12,4499 | 0 | radiosensible |
| Témoin lympho GM03798 | | | 15,7851 | 25,7 | radiorésistant |
| Témoin lympho GM03798 | | | 22,8777 | 32,27 | radiorésistant |
| Lympho 37792 muté BRCA 1 | | | 24,8657 | 18,7 | radiosensible |
| Lympho 40477 muté BRCA 1 | | | 20,5198 | 20,27 | radiosensible |

Pour les lymphoblastes du fait de la petite taille du cytoplasme par rapport au noyau, la réparation est plus rapide ce qui implique que les temps t1 et t2 sont compris dans un intervalle de temps allant de 0 à 30 min, intervalle dans lequel le maximum de cassures double brin (CDB) sont reconnues. A t1, la radiosensibilité du patient ne peut être déterminée de manière fiable car la quantité maximale de pATM dans le noyau n'est pas atteinte et donc ce temps ne peut rendre compte de la réparation notamment dans le cas de patients radiosensibles. A partir d'un temps t compris entre t1 fois 1,2 et t3, de préférence à t2, la radiosensibilité peut être déterminée de manière fiable car cela correspond à un temps ou au moins environ 30 % ou plus des CDB sont réparées chez un patient radiorésistant.

Chez les témoins, à t2=10 min on observe une activité kinase d'ATM prédominante dans le noyau (tableaux 7 et 8) ; cela correspond au schéma général pouvant être observé chez des sujets radiorésistants (voir les tableaux 7 et 8).

De la même façon chez un patient ayant une Chorée de Huntington (GM13515) il a été montré par immunofluorescence qu'il existait un retard dans le nucléoshuttling de la protéine ATM et ce par le biais d'une séquestration cytoplasmique par la protéine Huntington (Ferlazzo et al, « Mutations of the Huntington's Disease Protein Impact on the ATM-Dependent Signaling and Repair Pathways of the Radiation-Induced DNA Double-Strand Breaks: Corrective Effect of Statins and Bisphosphonates » paru dans Mol. Neurobiol. Vol. 49 p.1200-1211 en 2014). Ce retard a pu être mis en évidence aussi par le test selon l'invention test puisqu'à t2=10 min on n'observe pas d'activité kinase de la protéine ATM dans le noyau (voir les tableaux 7 et 8).

2.5 Les niveaux et méthodes d'analyse

Dans un mode de réalisation, tous les paramètres mesurés expérimentalement entrent dans la détermination des scores. En cas de conflit entre scores on répète certaines mesures ou détermination, ou on effectue d'autres essais expérimentaux de manière à ajouter d'autres mesures entrant dans la détermination des scores. Le niveau de certains paramètres seuls tels que le pATM $_{max\ nuc}$ suffit à établir un score et un diagnostic.

**Exemples**

L'invention est illustrée ci-dessous par des exemples qui cependant ne limitent pas l'invention. Ces exemples portent sur l'analyse de lignées cellulaires de patients permettant la détermination du grade de sévérité et du groupe de radiosensibilité auquel appartient le patient.

1. Préparation de l'échantillon cellulaire

Un échantillon sanguin d'un patient comportant des lymphocytes ou des lymphoblastes a été prélevé. Les lymphocytes ont été isolés à partir du sang total frais via la technique du ficoll connue de l'homme du métier et notamment présentée dans l'article de Bøyum, A. paru en 1968 intitulé « Isolation of mononuclear cells and granulocytes from human blood. » (Paper IV). Scand. J. Clin. Lab. Invest. 21 Suppl. 97, p.77-89.

La séparation des constituants du sang a été obtenue par centrifugation sur un coussin de Ficoll (polymère glucidique de masse molaire élevée). Après centrifugation pendant 20 min à 400g à température ambiante, les constituants du sang sont séparés par densité. Après élimination du surnageant contenant le plasma et les plaquettes, les lymphocytes ont été extraits de l'échantillon cellulaire, puis ont été lavés de façon à éliminer toute trace de Ficoll, qui est un polymère toxique pour les cellules.

Une partie de ces lymphocytes a été irradiée. L'autre partie n'a pas été irradiée, cette partie représentant l'état spontané (dose absorbée 0 Gy).

Un échantillon cellulaire de peau d'un patient a été prélevé par biopsie via la méthode de « punch dermatologique » connue de l'homme du métier. L'échantillon cellulaire a ensuite été placé dans du milieu DMEM+20% sérum de veau fétal stérile. L'échantillon cellulaire a ensuite été transféré sans délai dans un laboratoire spécialisé, afin que l'échantillon ne reste pas plus de 38 h à la température ambiante.

Dès réception, l'échantillon cellulaire issu de la biopsie a été établi sous la forme d'une lignée cellulaire amplifiable suivant une procédure bien connue des laboratoires de culture et de l'homme du métier : en utilisant notamment la dispersion trypsique les cellules sont à nouveau diluée dans du milieu renouvelé et ainsi de suite jusqu'à obtention du nombre de cellules souhaité. Après obtention d'un nombre de cellules suffisant, (généralement au bout d'une à trois semaines), les premières expériences ont été réalisées en utilisant le procédé selon l'invention. Les cellules ont été ensemencées sur lamelles de verre dans des boîtes de Pétri. Une partie de ces lamelles a été irradiée. L'autre partie n'a pas été irradiée, cette partie représentant l'état spontané (dose absorbée 0 Gy).

2. Irradiation de l'échantillon cellulaire

Les cellules de l'échantillon cellulaire ont été irradiées sur un irradiateur médical avec un accélérateur médical qui délivre des photons de 6 MV avec un débit de dose absorbée de 3 Gy min$^{-1}$ suivant une dosimétrie certifiée avec une dose absorbée D de 2 Gy.

3. Détermination de l'activité kinase après un temps t1 et un temps t2 de réparation post-irradiation avec une dose absorbée de 2Gy

La quantité de protéine pATM activée a été mesuré sur les cellules irradiées après un lapse de temps déterminé t (temps de réparation post-irradiation), à compter de l'arrêt de l'irradiation, fonction du type cellulaire étudié.

Échantillon de peau / fibroblastes : Ainsi, pour des fibroblastes, la quantité de protéines pATM a été mesuré à:

- t0 représentant l'état spontané non irradié,
- t1 représentant le temps au bout duquel le nombre de CDB reconnues atteint son maximum dans des cellules témoins issues de patients radiorésistants,
- et t2 représentant le temps au bout duquel environ 50 % des CDB sont réparées dans des fibroblastes témoins issus de patients radiorésistants.

Les effets de l'irradiation ont été observés à des temps précoces, i.e. 10 min (t1) et 60 min (t2) post irradiation.

Échantillon sanguin (lymphocytes ou lymphoblastes) : Ainsi, pour un échantillon sanguin, comportant notamment des lymphocytes ou pour des lymphoblastes, la quantité de protéines pATM a été mesurée à un instant :

- t1 représentant le temps au bout duquel le nombre de CDB reconnues atteint son maximum dans des cellules témoins issues de patients radiorésistants,
- et t2 représentant le temps au bout duquel 30 % des CDB sont réparées dans des lymphocytes ou lymphoblastes témoins issus de patients radiorésistants.

Les effets de l'irradiation ont été observés à des temps précoces, i.e. 5 min (t1) et 10 min (t2) post irradiation.

Un fractionnement subcellulaire afin de séparer les protéines cytoplasmiques des protéines nucléaires aux différents t0, t1 et t2 a été effectué sur les échantillons biologiques irradiés. Ce fractionnement a été réalisé en utilisant un Kit (PIERCE) selon la méthodologie décrite dans l'article de Trotter, K. W. and Archer, T. K., Molecular and Cellular Biology (2004) "Reconstitution of glucocorticoid receptor-dependent transcription in vivo ».

La présence des protéines dans chacune des fractions cytoplasmiques et nucléaires a été vérifiée par un test de type « Western blot » avec des anticorps spécifiques de chaque compartiment cytoplasmique (anti-tubuline ou anti-actine) et nucléaire (anti-topoisomérase).

Détermination du nombre de pATM activée par un test ELISA (Kit R&D systems et

Novateinbio) : La quantification des formes phosphorylées des protéines ATM (donc actives) dans chacune des fractions cytoplasmiques et nucléaires est réalisée par TEST ELISA (Kit) grâce à un anticorps spécifique anti-pATM coaté dans des plaques (R&D systems et Novateinbio). La quantité de pATM est ensuite analysée par luminescence à 450 nm grâce à un lecteur de plaques.

Les expérimentations ont été réalisées pour l'instant sur 3 lignées de lymphoblastes et 30 lignées de fibroblastes humains. Ces lignées ont été caractérisées en immunofluorescence pour le marquage H2AX et pATM (lignées issues de patients).

Détermination par spectrométrie de masse : L'isolation et la capture des protéines ATM dans chacune des fractions cytoplasmiques et nucléaires a été réalisée par immunoprécipitation grâce à un anticorps spécifique anti-ATM (AbCam).

L'activité enzymatique de la protéine ATM a ensuite été analysée via un test enzymatique de phosphorylation par la protéine kinase ATM active, d'une protéine cible ou substrat spécifique la protéine DNApK. L'ensemble de la réaction (protéine kinase ATM, substrat DNApK, ATP) a ensuite été digéré par une trypsine, puis séparé par chromatographie et analysé par spectrométrie de masse. Une analyse quantitative des différents éléments a été effectuée grâce à des peptides marqués permettant de détecter le nombre de substrats phosphorylés et non phosphorylés mais aussi la quantité de protéine ATM (normalisation des résultats).

1. Détermination de la radiosensibilité sur des lymphocytes ou lymphoblastes en fonction du dosage quantitatif des protéines pATM $_{max\,nuc}$ du test Elisa

La radiosensibilité du patient a pu être déterminée en fonction de la quantité de $pATM_{max\,nuc}$ et du seuil de 0,3 ng par million de cellules.

Si pour l'échantillon cellulaire $pATM_{max\,nuc}$ < 0,3 ng alors l'échantillon est radiosensible.

Dans cette formule $pATM_{max\,nuc}$ représente la quantité nucléaire maximale exprimée en ng de pATM par million de cellules.

Si pour l'échantillon cellulaire $pATM_{max\,nuc}$ ≥ 0,3 ng alors l'échantillon est radiorésistant.

2. Détermination de la radiosensibilité sur des fibroblastes en fonction du dosage quantitatif des protéines pATM max nuc du test Elisa

La radiosensibilité du patient a pu être déterminée en fonction de la quantité de $pATM_{max\,nuc}$ et du seuil de A ng par million de cellules.

Selon l'invention on considère que :

- l'échantillon est radiorésistant si pATM $_{max\,nuc}$ ≥ A ng par million de cellules,
- l'échantillon est radiosensible si pATM $_{max\,nuc}$ < A ng par million de cellules.

où $pATM_{max\,nuc}$ représente la quantité nucléaire maximale exprimée en ng de pATM par million de cellules et A est une valeur entière ou décimale comprise entre 0,1 et 15, de préférence entre 0,2 et 10 et encore plus préférentiellement entre 0,2 et 5.

Une méthode alternative consiste à déterminer la radiosensibilité de l'échantillon cellulaire de la manière suivante où on considère que :

- l'échantillon est radiorésistant si % pATM $_{max}$ ≥ B %

- l'échantillon est radiosensible si % pATM $_{max\ nuc}$ < B %

où

- % pATM$_{max\ nuc}$ représente la quantité nucléaire maximale en ng de pATM divisée par la quantité totale de protéines cellulaires de l'échantillon X 100, et
- B est une valeur entière ou décimale comprise entre 0,5x10$^{-4}$ et 5x10$^{-4}$.

pATM$_{max\ nuc}$ est la quantité nucléaire maximale (en ng) de pATM par million de cellules détectée par ELISA.

% pATM$_{max\ nuc}$ représente la quantité nucléaire maximale en ng de pATM divisée par la quantité totale de protéines cellulaires de l'échantillon X 100.

3. Détermination du grade de sévérité sur des fibroblastes par un dosage quantitatif des protéines pATM $_{max\ nuc}$ par la méthode du test Elisa

La quantité en ng de protéine pATM par million de cellules (pATM$_{maxnuc}$) dans les fibroblastes a été déterminée par l'emploi d'un test Elisa sur différents patients (cf tableau 9) dont un patient témoin dont les lignées cellulaires sont issues du Coriell Institute (AG11498 et GM02718). Les lignées A1 et A2 ont été établies à partir de biopsies de patients.

Tableau 9 : Valeurs maximales en ng de la protéine pATM par million de cellules (pATM$_{max\ nuc}$) dans les fibroblastes pour différents patients

| Lignée | pATM$_{max\ nuc}$ (max pATM/ millions de cellules) | max foci pATM | Grade CTCAE | statut de radiosensibilité |
|---|---|---|---|---|
| Témoin | 0,27 | 40 | 0-1 | radiorésistant |
| A1 | 0,105 | 23 | 2-3 | radiosensible |
| GM02718 | 0,124 | 26 | 2-3 | radiosensible |
| AG11498 | 0,19 | 25 | 2-3 | radiosensible |
| A2 | 0,069 | 20 | 4 | radiosensible |

Le grade CTCAE selon l'invention a pu être déterminé par application numérique de la formule du grade CTCAE présentée ci-dessous :

$$grade\ CTCAE = 5 - \frac{C}{10}\left(1 - \exp(-D * pATM_{max\ nuc})\right) \qquad (4)$$

où

- pATM$_{max\ nuc}$ représente la quantité nucléaire maximale en ng de pATM par million de cellules,
- C est une constante entière ou décimale qui représente la quantité maximale de foci pATM$_{max\ nuc}$ atteinte pour un échantillon radiorésistant, et qui appartient à l'intervalle [25 ,60] foci de préférence qui appartient à l'intervalle [30 ; 50] foci,
- D est une constante d'ajustement entière ou décimale qui appartient à l'intervalle [10 ,30], et qui correspond à l'inverse d'une quantité de pATM en ng par million de cellules.

**Revendications**

1. Procédé pour caractériser la radiosensibilité d'un échantillon cellulaire d'un patient envers un rayonnement ionisant, ledit échantillon cellulaire ayant été obtenu à partir de cellules prélevées sur un patient, dans lequel procédé:

(a) on isole des cellules nucléées cibles constituant l'échantillon cellulaire ;
(b) on irradie ledit échantillon cellulaire avant ou après l'étape (a) avec une dose absorbée D d'un rayonnement ionisant,
(c) on récupère les fractions cytoplasmiques et nucléaires de l'échantillon cellulaire ;

(d) on détermine sur les fractions cytoplasmiques et/ou nucléaires dudit échantillon cellulaire :

(d1) la quantité de substrat phosphorylé par la protéine ATM activée, qui est une protéine cible phosphorylée par la kinase ATM, à au moins un temps d'observation t compris entre t1 fois 1,2 et t3, de préférence à au moins un temps d'observation t2 après irradiation avec une dose absorbée D ; et/ou
(d2) l'activité kinase par la quantification de pATM phosphorylée à au moins un temps d'observation t sélectionné parmi t= t1, t2 ;

(e) on détermine au moins un paramètre sélectionné dans le groupe formé par :

- le grade de sévérité de la réaction tissulaire post-radiothérapie du patient selon la classification CTCAE, en utilisant au moins le nombre moyen pATM $_{max\,nuc}$ (t);
- la radiosensibilité de l'échantillon cellulaire en utilisant au moins le nombre moyen pATM $_{max\,nuc}$ (t);

et où

• t1 est une valeur fixe qui représente le temps au bout duquel le nombre de cassures double-brin (CDB) reconnues atteint son maximum dans des cellules témoins issues de patients radiorésistants ;
• t2 est une valeur fixe qui représente le temps au bout duquel environ 50 % des CDB sont réparées dans des fibroblastes témoins issus de patients radiorésistants et le temps au bout duquel 30 % des CDB sont réparées dans des lymphocytes ou lymphoblastes témoins issus de patients radiorésistants;
• t3 est une valeur fixe qui représente z fois t2, où z est compris entre 1,1 et 8, et de préférence compris entre 1,2 et 6.

2. Procédé selon la revendication 1, dans lequel à l'étape (d1) la quantité de substrat phosphorylée par la protéine ATM activée est déterminée par spectrométrie de masse.

3. Procédé selon la revendication 1 ou 2, dans lequel :

(i) on considère que l'échantillon cellulaire est radiorésistant, si à un instant d'observation t compris entre t1 fois 1,2 et t3, et préférentiellement à t2, la relation $qPP_{nuc} \geq qPP_{cyto}$ est valable,
(ii) on considère que l'échantillon est très radiosensible, si à un instant d'observation t compris entre t1 fois 1,2 et t3, et préférentiellement à t2, la relation $qPP_{cyto} > qPP_{nuc}$ est valable.

où

- $qPP_{cyto}$ correspond à la quantité de peptides phosphorylés dans la fraction cytoplasmique de l'échantillon cellulaire,
- $qPP_{nuc}$ correspond à la quantité de peptides phosphorylés dans la fraction nucléaire de l'échantillon cellulaire.

4. Procédé selon l'une quelconque des revendications 1 à 3, dans lequel la quantité de la protéine pATM activée est détectée dans le cytoplasme et le noyau cellulaire par un test ELISA.

5. Procédé selon l'une quelconque des revendications 1 à 4, dans lequel l'échantillon cellulaire est choisi parmi : un échantillon sanguin comprenant des lymphocytes ou des lymphoblastes et un échantillon issu d'une biopsie comprenant des fibroblastes.

6. Procédé selon l'une quelconque des revendications 1 à 5, dans lequel les dites-cellules nucléées cibles sont choisis parmi : des lymphoblastes, des lymphocytes et des fibroblastes.

7. Procédé selon l'une quelconque des revendications 1 à 6, dans lequel la radiosensibilité d'un échantillon cellulaire d'un patient envers un rayonnement ionisant est déterminée sur des fibroblastes issus de biopsie de patient et dans lequel

- on considère que l'échantillon est radiorésistant si pATM $_{max\,nuc}$ $\geq$ A ng par million de cellules,
- on considère que l'échantillon est radiosensible si pATM $_{max\,nuc}$ < A ng par million de cellules,

ou

- on considère que l'échantillon est radiorésistant si % pATM $_{max\,nuc} \geq$ B %
- on considère que l'échantillon est radiosensible si % pATM $_{max\,nuc}$ < B %

où

- pATM$_{max\,nuc}$ représente la quantité nucléaire maximale en ng de pATM par million de cellules,
- A est une valeur entière ou décimale comprise entre 0,1 et 15, de préférence entre 0,2 et 10 et encore plus préférentiellement entre 0,2 et 5,
- % pATM$_{max\,nuc}$ représente la quantité nucléaire maximale en ng de pATM divisée par la quantité totale de protéines cellulaires de l'échantillon X 100, et
- B est une valeur entière ou décimale comprise entre 0,5x10$^{-4}$ et 5x10$^{-4}$.

8. Procédé selon l'une quelconque des revendications 1 à 6, dans lequel la radiosensibilité d'un échantillon cellulaire d'un patient envers un rayonnement ionisant est déterminée sur des lymphoblastes et des lymphocytes issus de cellules sanguines et dans lequel

- on considère que l'échantillon est radiorésistant si pATM $_{max\,nuc} \geq$ 0,3 ng par million de cellules,
- on considère que l'échantillon est radiosensible si pATM $_{max\,nuc}$ < 0,3 ng par million de cellules,

où pATM$_{max\,nuc}$ représente la quantité nucléaire maximale en ng de pATM par million de cellules.

9. Procédé selon l'une quelconque des revendications 1 à 7 dans lequel on détermine le grade de sévérité de la réaction tissulaire post-radiothérapie selon la classification CTCAE selon la formule :

$$grade\ CTCAE = 5 - \frac{C}{10}(1 - \exp(-D * pATM_{max\,nuc}))$$

où

- pATM $_{max\,nuc}$ désigne la quantité nucléaire maximale en ng de pATM par million de cellules,
- C est une constante entière ou décimale qui représente la quantité maximale de foci pATM$_{max\,nuc}$ atteinte pour un échantillon radiorésistant, notamment observé par immunofluorescence, et qui appartient à l'intervalle [25 ; 60] foci, de préférence qui appartient à l'intervalle [30 ; 50] foci.
- D est une constante d'ajustement entière ou décimale qui appartient à l'intervalle [10 , 30] et qui correspond à l'inverse d'une quantité de pATM en ng par million de cellules.

10. Procédé selon l'une quelconque des revendications 1 à 9 dans lequel on détermine le grade CTCAE du patient à partir de la teneur en pATM$_{max\,nuc}$ (exprimée en ng par million de cellules dans des fibroblastes) selon au moins une des relations suivantes :

| Grades CTCAE | pATM$_{max\,nuc}$ |
|---|---|
| 0-1 | $\geq$0,25 |
| 2-3 | $\geq$0,07 et <0,25 |
| 4 | $\geq$0,025 et <0,07 |
| 5 | <0,025 |

**Patentansprüche**

1. Verfahren zum Charakterisieren der Strahlenempfindlichkeit einer Zellprobe eines Patienten gegenüber einer ionisierenden Strahlung, wobei die Zellprobe aus Zellen, die einem Patienten entnommen wurden, erhalten wurde, wobei in dem Verfahren:

(a) die kernhaltigen Zielzellen, die die Zellprobe bilden, isoliert werden;

(b) die Zellprobe vor oder nach Schritt (a) mit einer Energiedosis D einer ionisierenden Strahlung bestrahlt wird,

(c) die cytoplasmatischen und nukleären Fraktionen der Zellprobe gewonnen werden;

(d) anhand der cytoplasmatischen und/oder nukleären Fraktionen der Zellprobe Folgendes bestimmt wird:

(d1) die Menge des von dem aktivierten Protein ATM, das ein von der Kinase ATM phosphoryliertes Zielprotein ist, phosphorylierten Substrats zu mindestens einer Beobachtungszeit t zwischen t1 mal 1,2 und t3, vorzugsweise zu mindestens einer Beobachtungszeit t2 nach Bestrahlung mit einer Energiedosis D; und/oder

(d2) die Kinaseaktivität anhand der Quantifizierung von phosphoryliertem pATM zu mindestens einer zwischen t = t1, t2 ausgewählten Beobachtungszeit t;

(e) es wird mindestens ein Parameter bestimmt, der ausgewählt ist aus der Gruppe, die gebildet ist aus:

- dem Schweregrad der Gewebereaktion nach der Strahlentherapie des Patienten gemäß der CTCAE-Klassifikation anhand mindestens des Mittelwerts pATM $_{max\,nuc}$ (t);
- der Strahlenempfindlichkeit der Zellprobe anhand mindestens des Mittelwerts pATM $_{max\,nuc}$ (t);

und wobei

• t1 ein fester Wert ist, der die Zeit darstellt, nach der die Anzahl der festgestellten Doppelstrangbrüche (DSB) ihr Maximum in den von strahlenresistenten Patienten stammenden Kontrollzellen erreicht;
• t2 ein fester Wert ist, der die Zeit darstellt, nach der etwa 50 % der DSB in den Kontrollfibroblasten von strahlenresistenten Patienten repariert sind und die Zeit, nach der 30 % der DSB in den von strahlenresistenten Patienten stammenden Kontrolllymphozyten oder Kontrolllymphoblasten repariert sind;
• t3 ein fester Wert ist, der z mal t2 darstellt, wobei z zwischen 1,1 und 8 und vorzugsweise zwischen 1,2 und 6 liegt.

2. Verfahren nach Anspruch 1, wobei in Schritt (d1) die von dem aktivierten Protein ATM phosphorylierte Menge an Substrat durch Massenspektrometrie bestimmt wird.

3. Verfahren nach Anspruch 1 oder 2, wobei:

(i) davon ausgegangen wird, dass die Zellprobe strahlenresistent ist, wenn zu einem Beobachtungszeitpunkt t zwischen t1 mal 1,2 und t3 und vorzugsweise t2 das Verhältnis q$PP_{nuc}$ ≥ q$PP_{cyto}$ gültig ist,
(ii) davon ausgegangen wird, dass die Zellprobe sehr strahlenempfindlich ist, wenn zu einem Beobachtungszeitpunkt t zwischen t1 mal 1,2 und t3 und vorzugsweise t2 das Verhältnis q$PP_{cyto}$ > q$PP_{nuc}$ gültig ist.
wobei

- qPP$_{cyto}$ der Menge an phosphorylierten Peptiden in der cytoplasmatischen Fraktion der Zellprobe entspricht,
- qPP$_{nuc}$ der Menge an phosphorylierten Peptiden in der nukleären Fraktion der Zellprobe entspricht.

4. Verfahren nach einem der Ansprüche 1 bis 3, wobei die Menge des aktivierten Proteins pATM im Cytoplasma und im Zellkern durch einen ELISA-Test nachgewiesen wird.

5. Verfahren nach einem der Ansprüche 1 bis 4, wobei die Zellprobe ausgewählt ist aus: einer Blutprobe, die Lymphozyten oder Lymphoblasten enthält, und einer Biopsieprobe, die Fibroblasten enthält.

6. Verfahren nach einem der Ansprüche 1 bis 5, wobei die kernhaltigen Zielzellen ausgewählt sind aus: Lymphoblasten, Lymphozyten und Fibroblasten.

7. Verfahren nach einem der Ansprüche 1 bis 6, wobei die Strahlenempfindlichkeit einer Zellprobe eines Patienten gegenüber einer ionisierenden Strahlung anhand von Fibroblasten, die aus einer Biopsie bei einem Patienten stammen, bestimmt wird und wobei

- davon ausgegangen wird, dass die Probe strahlenresistent ist, wenn pATM $_{max\,nuc}$ ≥ A ng pro Million Zellen ist,
- davon ausgegangen wird, dass die Probe strahlenempfindlich ist, wenn pATM $_{max\,nuc}$ < A ng pro Million Zellen ist,
oder
- davon ausgegangen wird, dass die Probe strahlenresistent ist, wenn % pATM $_{max\ nuc}$ ≥ B % ist
- davon ausgegangen wird, dass die Probe strahlenempfindlich ist, wenn % pATM $_{max\ nuc}$ < B % ist,
wobei
- pATM$_{max\ nuc}$ die maximale nukleäre Menge in ng pATM pro Million Zellen darstellt,
- A ein ganzzahliger oder dezimaler Wert zwischen 0,1 und 15, vorzugsweise zwischen 0,2 und 10 und noch bevorzugter zwischen 0,2 und 5 ist,
- % pATM$_{max\ nuc}$ die maximale nukleäre Menge in ng pATM geteilt durch die Gesamtmenge der Zellproteine der Probe X 100 darstellt und
- B ein ganzzahliger oder ein dezimaler Wert zwischen $0,5 \times 10^{-4}$ und $5 \times 10^{4}$ ist.

8.  Verfahren nach einem der Ansprüche 1 bis 6, wobei die Strahlenempfindlichkeit einer Zellprobe eines Patienten gegenüber einer ionisierenden Strahlung anhand von Lymphoblasten und Lymphozyten, die aus Blutzellen stammen, bestimmt wird und wobei

    - davon ausgegangen wird, dass die Probe strahlenresistent ist, wenn pATM $_{max\ nuc}$ ≥ 0,3 ng pro Million Zellen ist,
    - davon ausgegangen wird, dass die Probe strahlenempfindlich ist, wenn pATM $_{max\ nuc}$ < 0,3 ng pro Million Zellen ist,

    wobei pATM$_{max\ nuc}$ die maximale nukleäre Menge in ng pATM pro Million Zellen darstellt.

9.  Verfahren nach einem der Ansprüche 1 bis 7, wobei der Schweregrad der Gewebereaktion nach der Strahlentherapie gemäß der CTCAE-Klassifikation nach der folgenden Formel bestimmt wird:

$$CTCAE - Grad \ = \ 5 \ - \frac{C}{10}\bigl(1 \ - \exp(-D \ * \ pATM_{\max nuc})\bigr)$$

    wobei

    - pATM$_{max\ nuc}$ die maximale nukleäre Menge in ng pATM pro Million Zellen bezeichnet,
    - C eine ganzzahlige oder dezimale Konstante ist, die die für eine strahlenresistente Probe erreichte maximale Menge an pATM$_{max\ nuc}$-Foci - hauptsächlich bei Immunfluoreszenz beobachtet - darstellt, und die zum Foci-Intervall [25; 60] gehört, die vorzugsweise zum Foci-Intervall [30; 50] gehört.
    - D eine ganzzahlige oder dezimale Anpassungskonstante ist, die zum Intervall [10, 30] gehört und die der Umkehrung einer Menge an pATM in ng pro Million Zellen entspricht.

10. Verfahren nach einem der Ansprüche 1 bis 9, wobei der CTCAE-Grad des Patienten anhand des Gehalts an pATM$_{max\ nuc}$(ausgedrückt in ng pro Million Zellen in den Fibroblasten) nach mindestens einem der folgenden Verhältnisse bestimmt wird:

| CTCAE-Grade | pATM$_{max\ nuc}$ |
|---|---|
| 0-1 | ≥ 0,25 |
| 2-3 | ≥ 0,07 und < 0,25 |
| 4 | ≥ 0,025 und < 0,07 |
| 5 | < 0,025 |

**Claims**

1.  Method for characterizing the radiosensitivity of a patient's cell sample to ionizing radiation, said cell sample having been obtained from cells taken from a patient, in which method:

23

(a) target nucleated cells constituting the cell sample are isolated;

(b) said cell sample is irradiated before or after step (a) with an absorbed dose D of ionizing radiation;

(c) the cytoplasmic and nuclear fractions are recovered from the cell sample;

(d) the following are determined on the cytoplasmic and/or nuclear fractions of said cell sample:

(d1) the quantity of substrate phosphorylated by the activated ATM protein, which is a target protein phosphorylated by the ATM kinase, at at least one observation time t between t1 times 1.2 and t3, preferably at at least one observation time t2 after irradiation with an absorbed dose D; and/or

(d2) the kinase activity by quantifying phosphorylated pATM at at least one observation time t selected from t = t1, t2;

(e) at least one parameter is determined, selected from the group formed by:

- the degree of severity of the patient's post-radiotherapy tissue reaction according to CTCAE classification, using at least the mean number $pATM_{max\ nuc}$ (t);
- the radiosensitivity of the cell sample, using at least the mean number $pATM_{max\ nuc}$ (t);

and/or

• t1 is a fixed value which represents the time after which the number of double-strand breaks (DSB) recognized reaches its maximum in control cells obtained from radioresistant patients;

• t2 is a fixed value which represents the time after which approximately 50% of the DSBs are repaired in control fibroblasts obtained from radioresistant patients and the time after which 30% of the DSBs are repaired in control lymphocytes or lymphoblasts obtained from radioresistant patients;

• t3 is a fixed value which represents z times t2, where z is between 1.1 and 8, and preferably between 1.2 and 6.

2. Method according to claim 1, wherein in step (d1) the quantity of substrate phosphorylated by the activated ATM protein is determined by mass spectrometry.

3. Method according to either claim 1 or claim 2, wherein:

(i) the cell sample is considered to be radioresistant if, at an observation instant t between t1 times 1.2 and t3, and preferably at t2, the relationship $qPP_{nuc} \geq qPP_{cyto}$ is valid,

(ii) the sample is considered to be very radiosensitive if, at an observation instant t between t1 times 1.2 and t3, and preferably at t2, the relationship $qPP_{cyto} > qPP_{nuc}$ is valid,

where

- $qPP_{cyto}$ corresponds to the quantity of phosphorylated peptides in the cytoplasmic fraction of the cell sample,
- $qPP_{nuc}$ corresponds to the quantity of phosphorylated peptides in the nuclear fraction of the cell sample.

4. Method according to any of claims 1 to 3, wherein the quantity of the activated pATM protein is detected in the cytoplasm and the cell nucleus by an ELISA test.

5. Method according to any of claims 1 to 4, wherein the cell sample is selected from: a blood sample comprising lymphocytes or lymphoblasts, and a sample obtained from a biopsy comprising fibroblasts.

6. Method according to any of claims 1 to 5, wherein said target nucleated cells are selected from: lymphoblasts, lymphocytes, and fibroblasts.

7. Method according to any of claims 1 to 6, wherein the radiosensitivity of a patient's cell sample to ionizing radiation is determined on fibroblasts obtained from patient biopsies and wherein

- the sample is considered to be radioresistant if $pATM_{max\ nuc} \geq A$ ng per million cells,
- the sample is considered to be radiosensitive if $pATM_{max\ nuc} < A$ ng per million cells,

or

- the sample is considered to be radioresistant if % $pATM_{maxnuc} \geq B\%$

- the sample is considered to be radiosensitive if % $pATM_{max\ nuc}$ < B% where
- $pATM_{max\ nuc}$ represents the maximum nuclear quantity in ng of pATM per million cells,
- A is an integer or decimal value between 0.1 and 15, preferably between 0.2 and 10, and even more preferably between 0.2 and 5,
- % $pATM_{max\ nuc}$ represents the maximum nuclear quantity in ng of pATM divided by the total quantity of cellular protein in the sample X 100, and
- B is an integer or decimal value between $0.5 \times 10^{-4}$ and $5 \times 10^{-4}$.

8. Method according to any of claims 1 to 6, wherein the radiosensitivity of a patient's cell sample to ionizing radiation is determined on lymphoblasts and lymphocytes obtained from blood cells and wherein

- the sample is considered to be radioresistant if $pATM_{max\ nuc} \geq 0.3$ ng per million cells,
- the sample is considered to be radiosensitive if $pATM_{max\ nuc} < 0.3$ ng per million cells,
where $pATM_{max\ nuc}$ represents the maximum nuclear quantity in ng of pATM per million cells.

9. Method according to any of claims 1 to 7, wherein the degree of severity of the post-radiotherapy tissue reaction is determined according to CTCAE classification according to the following formula:

$$CTCAE\ grade = 5 - \frac{C}{10}\left(1 - \exp(-D * pATM_{\max nuc})\right)$$

where

- $pATM_{max\ nuc}$ denotes the maximum nuclear quantity in ng of pATM per million cells,
- C is an integer or decimal constant which represents the maximum quantity of $pATM_{max\ nuc}$ foci reached for a radioresistant sample, in particular observed by immunofluorescence, and which belongs to the interval [25; 60] foci, preferably which belongs to the interval [30; 50] foci,
- D is an integer or decimal adjustment constant which belongs to the interval [10, 30] and which corresponds to the inverse of a quantity of pATM in ng per million cells.

10. Method according to any of claims 1 to 9, wherein the patient's CTCAE grade is determined from the $pATM_{max\ nuc}$ content (expressed in ng per million cells in fibroblasts) according to at least one of the following relationships:

| CTCAE grades | $pATM_{max\ nuc}$ |
|---|---|
| 0-1 | $\geq 0.25$ |
| 2-3 | $\geq 0.07$ and $< 0.25$ |
| 4 | $\geq 0.025$ and $< 0.07$ |
| 5 | $< 0.025$ |

Figure 1

Figure 2

Figure 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2015121596 A1

### Littérature non-brevet citée dans la description

- **N. FORAY et al.** A subset of A TM- and ATR-dependent phosphorylation events requires the BRCA1 protein. *The EMBO Journal,* 2003, vol. 22 (11), 2860-2871
- **JOUBERT et al.** DNA double-strand break repair defects in syndromes associated with acute radiation response ; At least two different assays to predict intrinsic radiosensitivity ?. *Int. J. Radiat. Biol.,* 2008, vol. 84 (2), 107-125
- **GATELY et al.** Characterization of ATM Expression, Localization, and Associated DNA-dependent Protein Kinase Activity. *Molecular biology of the cell,* 1998
- **KHALIL et al.** ATM in focus: A damage sensor and cancer target. *biodiscovery,* 2012
- **BHATTI et al.** ATM protein kinase: the linchpin of cellular defenses to stress. *Cellular and Molecular Life Science,* 2011
- **KHORONENKOVA ; DIANOV.** ATM prevents DSB formation by coordinating SSB repair and cell cycle progression. *PNAS,* 2014
- **BØYUM, A.** Isolation of mononuclear cells and granulocytes from human blood. *Scand. J. Clin. Lab. Invest.,* 1968, vol. 21 (97), 77-89
- **TROTTER, K. W. ; ARCHER, T. K.** Reconstitution of glucocorticoid receptor-dependent transcription in vivo. *Molecular and Cellular Biology,* 2004
- Critères d'évaluation de la morbidité selon la classification du National Cancer Institute. *CTCAE,* 2006
- **FERLAZZO et al.** Mutations of the Huntington's Disease Protein Impact on the ATM-Dependent Signaling and Repair Pathways of the Radiation-Induced DNA Double-Strand Breaks: Corrective Effect of Statins and Bisphosphonates. *Mol. Neurobiol.,* 2014, vol. 49, 1200-1211